# FASCICULE DE BREVET EUROPEEN

(11) **EP 1 084 246 B1**
(45) Date de publication et mention de la délivrance du brevet: **15.09.2004**
(21) Numéro de dépôt: 99950801.3
(22) Date de dépôt: 20.10.1999
(51) Int. Cl.: C12N 15/13, C12N 5/10, A61K 48/00, A61K 35/14, A61P 31/12, A61P 35/00, C07K 16/28

(54) **MATERIEL BIOLOGIQUE POUR LA PREPARATION DE COMPOSITIONS PHARMACEUTIQUES DESTINEES AU TRAITEMENT DE MAMMIFERES**
BIOLOGISCHES MATERIAL ZUR HERSTELLUNG PHARMAZEUTISCHER ZUSAMMENSETZUNGEN ZUR BEHANDLUNG VON SÄUGETIEREN
BIOLOGICAL MATERIAL FOR PREPARING PHARMACEUTICAL COMPOSITIONS FOR TREATING MAMMALS

(30) Priorité: 22.10.1998 FR 9813279
(43) Date de publication de la demande: 21.03.2001
(73) Titulaire: TRANSGENE S.A., 67000 Strasbourg (FR)
(72) Inventeur: ACRES, Bruce, F-67000 Strasbourg (FR); PAUL, Stéphane, F-67000 Strasbourg (FR)
(74) Mandataire: Ahner, Francis
(86) Numéro de dépôt international: PCT/FR1999/002551
(87) Numéro de publication internationale: WO 2000/024896

(56) Documents cités:
- WO-A-97/02355
- FR-A- 2 758 569
- S. DESSUREAULT ET AL.: "Allogeneic lymphocyte responses to B7-1 expressing human cancer cell lines." JOURNAL OF SURGICAL RESEARCH, vol. 64, no. 1, 15 juillet 1996 (1996-07-15), pages 42-48, XP002113685 San Diego, CA, tats-Unis
- A. GUARINI ET AL.: "IL-2 gene-transduced human HLA-A2 melanoma cells can generate a specific antitumor cytotoxic T-lymphocyte response." CYTOKINES AND MOLECULAR THERAPY, vol. 1, no. 1, mars 1995 (1995-03), pages 57-64, XP002113686 Londres, Grande-Bretagne

## Description

La présente invention concerne le domaine de la thérapie génique appliquée à l'immunothérapie spécifique, plus particulièrement dans le cadre de traitements de maladies dont l'agent responsable est un organisme pathogène, tel que notamment un agent bactérien, parasitaire ou viral, ou dans le cadre du traitement du cancer. Plus particulièrement, l'invention concerne le transfert dans des cellules tumorales ou infectées par un agent pathogène, de séquences d'acide nucléique codant pour tout ou partie d'anticorps de sorte que les cellules génétiquement modifiées par ces séquences d'acide nucléique expriment à leur surface lesdits anticorps, et plus particulièrement des anticorps capables de se fixer à un polypeptide présent à la surface d'une cellule effectrice cytotoxique ou d'un lymphocyte T helper et impliqué dans le procédé d'activation d'une telle cellule.

Depuis longtemps, la thérapie génique a été proposée pour corriger les désordres observés dans le cadre des maladies génétiques. Ces maladies s'expliquent en particulier par un disfonctionnement de l'expression de gènes spécifiques ou par l'expression de polypeptides mutés non fonctionnels dans au moins un type cellulaire. La thérapie génique consiste dans ce cas à transférer dans des cellules cibles spécifiques extraites puis réintroduites dans le corps humain, ou directement dans les organes affectés, l'information génétique capable de corriger le défaut observé. Il pourra s'agir par exemple du gène codant pour la protéine CFTR dans le cas de la mucoviscidose ou du gène codant pour la dystrophine dans le cas de la myopathie de Duchenne. Dans le cadre de cette approche, l'information génétique est introduite soit *in vitro* dans une cellule extraite de l'organe, la cellule modifiée étant ensuite réintroduite dans l'organisme (procédé *ex vivo*), soit directement *in vivo* dans le tissu approprié. De nombreuses publications décrivent la mise en oeuvre de protocoles de thérapie génique afin d'obtenir dans les cellules cibles l'expression d'une protéine présentant un intérêt thérapeutique par introduction de l'information génétique correspondante.

Toutefois, l'intérêt de ce type de thérapie ne se borne pas au traitement des affections purement génétiques et peut également permettre l'élimination de tumeurs, ou d'agents pathogènes, tels que les agents bactériens ou viraux, ou de cellules infectées par de tels agents pathogènes, ou à défaut de retarder leur progression.

La réponse immune dirigée contre un antigène spécifique peut être divisée en deux catégories distinctes, l'une mettant en jeu les anticorps (réponse immune de type humoral), l'autre les cellules effectrices cytotoxiques telles que par exemple les macrophages, les lymphocytes cytotoxiques (CTL) ou les cellules tueuses (NK) ainsi que les lymphocytes T helper, notamment les LTCD4 (réponse immune de type cellulaire). Plus particulièrement, les deux types de réponses se distinguent en ce que les anticorps reconnaissent les antigènes sous leur forme tridimensionnelle alors que les lymphocytes T, par exemple, reconnaissent des portions peptidiques desdits antigènes, associés à des glycoprotéines codées par les gènes du complexe majeur d'histocompatibilité (ou CMH), notamment les gènes du complexe majeur d'histocompatibilité de type I qui sont exprimés de façon ubiquitaire à la surface des cellules ou les gènes du complexe majeur d'histocompatibilité de type II qui sont exprimés de façon spécifique à la surface des cellules impliquées dans la présentation des antigènes (APC).

Selon un premier aspect, la réponse immune de type cellulaire est caractérisée en ce que les cellules T de type CD4+ (cellules T «helper»), suite à un phénomène d'activation bien connu (pour une revue voir Alberola-Ila, 1997, Annu. Rev. Immunol., 15, 125-154) produisent des cytokines qui à leur tour induisent la prolifération de cellules APC capables de produire lesdites cytokines ; la différenciation cellulaire des lymphocytes B capables de produire des anticorps spécifiques ; et la stimulation des lymphocytes T cytotoxiques (CTL). Selon un second aspect de la réponse immune cellulaire, les cellules effectrices cytotoxiques telles que par exemple les lymphocytes de type CD8+ (CTL) sont activés a) après interaction avec des peptides antigéniques fixés sur et présentés par les glycoprotéines portées par les cellules ubiquitaires et codées par les gènes appartenant au système CMH I, et b) éventuellement par les cytokines produites par les CD4+. Les CTL ainsi activés sont alors capables de détruire les cellules exprimant ledit peptide antigénique.

Dans le cas particulier des cancers, Hellstrom et al., (1969, Adv. Cancer Res. 12, 167-223) ont montré que la défense de l'organisme à l'égard des tumeurs repose tout particulièrement sur la réponse immunitaire mettant en jeu les lymphocytes T, notamment les lymphocytes T cytotoxiques. Toutefois, de nombreux travaux ont montré que la plupart de ces effecteurs immunitaires, spécifiques ou non, sont inefficaces pour permettre l'élimination ou l'arrêt de progression d'une tumeur. C'est la raison pour laquelle il est souhaitable de disposer d'une méthode de stimulation de la réponse immune dirigée contre les tumeurs, et plus particulièrement de la réponse faisant intervenir les lymphocytes cytotoxiques CTL, afin de disposer de méthodes de prévention ou de traitement des états cancéreux plus efficaces. De manière identique, il a été montré que le système immunitaire est souvent inefficace dans le cas d'infections, virales notamment, voir par exemple le cas des infections dues au VIH (Virus de l'Immunodéficience Humaine).

Selon une première alternative, il a été proposé d'adapter les procédés de thérapie génique déjà bien connus et de transférer dans les cellules cibles, plus particulièrement les cellules cancéreuses, des gènes immunostimulateurs (immunothérapie) susceptibles d'induire ou d'activer une réponse immune à médiation cellulaire à l'égard de la tumeur, de gènes codant pour les cytokines (Colombo et al., 1994, Immunology Today, 15, 48-51), de gènes cytotoxiques conférant une toxicité aux cellules les exprimant, par exemple le gène *tk* du virus Herpes Simplex de type l (HSV-1), ou d'anti-oncogènes, tels que par exemple le gène associe au rétinoblastome ou p53, ou de polynucléotides capables d' inhiber l'activité d'un oncogène, tels que par exemple les molécules antisens ou les ribozymes capables de dégrader les ARN messagers spécifiques des oncogènes. Cependant, dans la majorité des cas, les cellules ainsi modifiées manquent de spécificité vis-à-vis de la tumeur et ne permettent pas une approche thérapeutique satisfaisante.

Une autre approche a également été proposée alliant les avantages de la thérapie génique et la mise en oeuvre d'anticorps spécifiques. Au cours des dernières années de nombreux antigènes tumoraux ont été caractérisés qui ont plus particulièrement permis l'identification d'anticorps, notamment d'anticorps monoclonaux spécifiques de ces antigènes. Par ailleurs, la production *in vitro* d'anticorps, de fragments d'anticorps ou de dérivés d'anticorps tels que les anticorps chimères, par génie génétique, dans des cellules eucaryotes a été décrite (EP 120 694 ou EP 125 023). Ainsi, de nombreuses stratégies thérapeutiques ont été proposées, par exemple pour le traitement ou la prévention de lymphomes B (Yefenof et al., 1993, Current Opinion in Immunology,5, 740-744), reposant sur l'administration au patient d'anticorps thérapeutiques ciblant des antigènes tumoraux afin de neutraliser l'agent causal de la maladie. Malheureusement, ces anticorps bien que très utiles pour la détection et le diagnostic des cancers se sont révélés non satisfaisants d'un point de vue thérapeutique car ils conduisent, par exemple, à des réactions immunes limitées, dirigées uniquement à l'encontre d'épitopes immunodominants ou contre des antigènes présentant une grande variabilité.

La demande de brevet internationale (WO 94/29446) décrit l'expression intracellulaire de séquences d'ADN codant pour des anticorps. Cette approche permet d'envisager une thérapie génique reposant sur le ciblage de composants cellulaires non accessibles par les méthodes de vaccination et se caractérise en ce que l'approche décrite n'implique pas le développement d'une réponse immunitaire, agit intracellulairement et par conséquent ne permet pas le traitement efficace de tumeurs.

La demande de brevet internationale (WO 98/31808) concerne au contraire l'expression *in vivo* de gènes codant pour des anticorps ou des fragments d'anticorps par des cellules capables de sécréter lesdits anticorps dans la circulation sanguine du mammifère porteur des cellules génétiquement modifiées par le gène codant pour l'anticorps. L'avantage de cette invention repose sur la possibilité de maintenir sur le long terme un niveau basal d'anticorps dans le patient traité.

Nous avons maintenant montré qu'il est possible de rediriger la réponse immunitaire cellulaire en exprimant à la surface de cellules cibles, notamment tumorales ou infectées par un agent pathogène, tout ou partie d'un anticorps capable de se fixer à un polypeptide présent à la surface de cellules effectrices cytotoxiques ou de lymphocytes T helper. Plus particulièrement, nous avons montré que selon la présente invention, ces cellules cibles génétiquement modifiées permettent de diriger l'activation des cellules effectrices cytotoxiques ou de lymphocytes T helper, d'augmenter l'effet cytotoxique de ces cellules, notamment activées, à l'égard des cellules cibles, de dynamiser la réponse immune à médiation cellulaire se produisant naturellement à l'égard des cellules tumorales ou infectées, génétiquement modifiées ou non. Ceci peut ainsi se traduire par la lyse et l'élimination desdites cellules cibles, des cellules voisines et à terme par l'élimination de la tumeur ou de l'infection.

La présente invention concerne en premier lieu un matériel biologique pour la préparation de compositions pharmaceutiques destinées au traitement de mammifères comprenant :
- soit au moins une séquence d'acide nucléique contenant au moins un gène d'intérêt thérapeutique et des éléments assurant l'expression dudit gène *in vivo* dans des cellules cibles destinées à être génétiquement modifiées par ladite séquence d'acide nucléique;
- soit au moins une cellule de mammifère ne produisant pas naturellement des anticorps et génétiquement modifiée in vitro par au moins une séquence d'acide nucléique précédente,
caractérisé en ce que ledit gène d'intérêt thérapeutique code pour tout ou partie d'un anticorps qui sera exprimé à la surface de ladite cellule de mammifère et en ce que ledit anticorps est capable de se fixer à un polypeptide présent à la surface d'une cellule effectrice cytotoxique ou d'un lymphocyte T helper, et impliqué dans le procédé d'activation d'une telle cellule.

Par «séquence d'acide nucléique», on entend désigner un fragment d'ADN et/ou d'ARN, double brin ou simple brin, linéaire ou circulaire, naturel isolé ou de synthèse, désignant un enchaînement précis de nucléotides, modifiés ou non, permettant de définir un fragment ou une région d'un acide nucléique sans limitation de taille. Selon un mode de réalisation préféré, il s'agit d'un acide nucléique choisi parmi le groupe consistant en un cADN ; un ADN génomique ; un ADN plasmidique ; un ARN messager.

Selon l'invention, ladite «séquence d'acide nucléique» comprend au moins un «gène d'intérêt thérapeutique» et des éléments d'expression dudit gène d'intérêt. Un tel «gène d'intérêt thérapeutique» code notamment pour tout ou partie d'un anticorps transmembranaire natif, ou pour un dérivé d'un tel anticorps, pour autant que ledit anticorps, fragment ou dérivé d'anticorps soit exprimé à la surface de la cellule cible de mammifère génétiquement modifiée et en ce que ledit anticorps est capable de se fixer à un polypeptide présent à la surface d'une cellule effectrice cytotoxique ou d'un lymphocyte T helper et impliqué dans le procédé d'activation d'une telle cellule. Plus particulièrement, par «fragment» d'anticorps on entend désigner les fragments F(ab)₂, Fab', Fab, sFv (Blazar et al., 1997, Journal of Immnunology, 159, 5821-5833 ; Bird et al., 1988, Science, 242, 423-426) d'un anticorps natif et par «dérivé» par exemple un dérivé chimérique d'un tel anticorps (voir par exemple les chimères les anticoprs anti CD3 Souris/Homme dans Arakawa et al., 1996, J. Biochem., 120, 657-662 ou les immunotoxines telles que sFv-toxine de Chaudary et al., 1989, Nature 339, 394-397). Par «anticorps transmembranaire» on entend désigner un anticorps dont au moins la région fonctionnelle capable de reconnaître et de se fixer à son antigène spécifique est exprimée à la surface des cellules cibles pour permettre lesdites reconnaissance et fixation. Plus particulièrement, les anticorps selon la présente invention consistent en des polypeptides de fusion comprenant les amino acides définissant ladite région fonctionnelle et une séquence d'amino acides (polypeptide transmembranaire) permettant l'ancrage au sein de la double couche lipidique membranaire de la cellule cible ou à la surface externe de cette bi-couche. Les séquences nucléiques codant pour de nombreux polypeptides transmembranaires sont décrites dans la littérature. Selon un cas préféré de l'invention, ledit polypeptide transmembranaire est sélectionné parmi le groupe consistant en une glycoprotéine, une lipoprotéine, en un récepteur tel que tout ou partie des complexes évoqués plus loin dans la demande (CD4, Fc, gp160 du VIH par exemple), et plus particulièrement parmi le groupe consistant en la glycoprotéine du virus de la rage (demande de brevet français n° FR 97 09152), le CD4 (Weijtens et al., 1998, Gene Therapy, 5, 1195-1203), la gp160, le Fc. Selon un cas tout à fait avantageux, la séquence d'acide nucléique codant pour la chaîne lourde de l'anticorps sera fusionnée avec la séquence d'acide nucléique codant pour undit polypeptide transmembranaire.

Par «éléments assurant l'expression dudit gène *in vivo*», on entend désigner les éléments nécessaires afin d'assurer l'expression dudit gène après son transfert dans une cellule cible. Il s'agit notamment des séquences promotrices et/ou des séquences de régulation efficaces dans ladite cellule, et éventuellement les séquences requises pour permettre l'expression à la surface des cellules cibles dudit polypeptide. Le promoteur utilisé peut être un promoteur viral, ubiquitaire ou spécifique de tissu ou encore un promoteur synthétique. A titre d'exemple, on mentionnera les promoteurs tels que les promoteurs des virus RSV. (Rous Sarcoma Virus), MPSV, SV40 (Simian Virus), CMV (Cytomegalovirus) ou du virus de la vaccine, les promoteurs du gène codant pour la créatine kinase musculaire, pour l'actine, pour le surfactant pulmonaire. Il est en outre possible de choisir une séquence promotrice spécifique d'un type cellulaire donné, ou activable dans des conditions définies. La littérature procure un grand nombre d'informations relatives à de telles séquences promotrices. Par ailleurs, ledit acide nucléique peut compendre au moins deux séquences, identiques ou différentes, présentant une activité de promoteur transcriptionnel et/ou au moins deux gènes, identiques ou différents, situés l'un par rapport à l'autre de manière contiguë, éloignée, dans le même sens ou en sens inverse, pour autant que la fonction de promoteur transcriptionnel ou la transcription desdits gènes ne soit pas affectée. De même, dans ce type de construction d'acide nucléique, il est possible d'introduire des séquences nucléiques «neutres» ou introns qui ne nuisent pas la transcription et sont épissées avant l'étape de traduction. De telles séquences et leurs utilisations sont décrites dans la littérature (WO 94/29471). Ledit acide nucléique pourra également renfermer des séquences requises pour le transport intracellulaire, pour la réplication et/ou pour l'intégration pour la transcription ou la traduction. De telles séquences sont bien connues de l'homme de l'art. Par ailleurs, les acides nucléiques utilisables selon la présente invention peuvent également être des acides nucléiques modifiés de sorte qu'il ne leur est pas possible de s'intégrer dans le génome de la cellule cible ou des acides nucléiques stabilisés à l'aide d'agents, tels que par exemple la spermine, qui en tant que tels n'ont pas d'effet sur l'efficacité de la transfection.

Selon un premier mode de réalisation de l'invention, la séquence d'acide nucléique est une séquence d'ADN ou ARN nue, c'est à dire libre de tout composé facilitant son introduction dans les cellules (transfert de séquence d'acide nucléique). Toutefois, afin de favoriser son introduction dans les cellules cibles afin d'obtenir les cellules génétiquement modifiées de l'invention, cette séquence d'acide nucléique peut être sous la forme d'un vecteur, et plus particulièrement sous la forme d'un vecteur viral tel que par exemple un vecteur adénoviral, rétroviral, un vecteur dérivé d'un poxvirus, notamment dérivé du virus de la vaccine ou du Modifed Virus Ankara (MVA) ou d'un vecteur non-viral tel que par exemple un vecteur consistant en au moins une dite séquence d'acide nucléique complexée ou conjuguée à au moins une molécule ou substance porteuse sélectionnée parmi le groupe consistant en un amphiphile cationique, notamment un lipide cationique, un polymère cationique ou neutry un composé polaire protique notamment choisi parmi le propylène glycol, le polyéthylène glycol, le glycérol, l'éthanol, la 1-methyl L -2-pyrrolidone ou leurs dérivés, et un composé polaire aprotique notamment choisi parmi le diméthylsulfoxide (DMSO), le diéthylsulfoxide, le di-n-propylsulfoxide, le diméthylsulfone, le sulfolane, la diméthylformamide, le diméthylacetamide, la tetraméthylurée, l'acétonitrile ou leurs dérivés.

Par ailleurs, de tels vecteurs peuvent en outre comprendre des éléments de ciblage pouvant permettre de diriger le transfert de séquence d'acide nucléique vers certains types cellulaires ou certains tissus particuliers (cellules tumorales, cellules de l'épithélium pulmonaire, cellule hématopoïétique, cellule musculaire, cellule nerveuse...). Ils peuvent également permettre de diriger le transfert d'une substance active vers certains compartiments intracellulaires préférés tels que le noyau et les mitochondries, par exemple. Il peut en outre s'agir d'éléments facilitant la pénétration à l'intérieur de la cellule ou la lyse des endosomes. De tels éléments de ciblage sont largement décrits dans la littérature. Il peut par exemple s'agir de tout ou partie de lectines, de peptides, notamment le peptide JTS-1 (voir demande de brevet WO 94/40958), d'oligonucléotides, de lipides, d'hormones, de vitamines, d'antigènes, d'anticorps, de ligands spécifiques de récepteurs membranaires, de ligands susceptibles de réagir avec un anti-ligand, de peptides fusogèniques, de peptides de localisation nucléaire, ou d'une combinaison de tels composés. En particulier, il peut s'agir de résidus galactosyl permettant de cibler le récepteur des asialoglycoprotéines à la surface des cellules hépatiques, de ligands pouvant interagir avec des récepteurs tels que des récepteurs de facteurs de croissance, des récepteur de cytokines, de lectines, de protéines d'adhésion, il peut également s'agir d'un fragment d'anticorps tel que le fragment Fab, d'un peptide fusogénique INF-7 dérivé de la sous unité HA-2 de l'hémagglutinine du virus influenza (Plank et al., 1994, J. Biol. Chem. 269, 12918-12924), d'un signal de localisation nucléaire dérivé de l'antigène T du virus SV40 ou de la protéine EBNA-1 du virus Epstein Barr.

Selon l'invention, l'anticorps exprimé à la surface des cellules cibles est capable de se fixer à un polypeptide présent à la surface d'une cellule effectrice cytotoxique ou d'un lymphocyte T helper, notamment un lymphocyte T helper CD4, et impliqué dans le procédé d'activation d'une telle cellule, et plus particulièrement à un récepteur directement impliqué dans un tel procédé. Comme cela est décrit précédemment, ce phénomène d'activation des cellules effectrices cytotoxiques ou de lymphocytes T helper est un élément déterminant de la réaction immunitaire à médiation cellulaire. Toutefois, il convient de remarquer que dans le cadre de la mise en oeuvre de la présente invention, il n'est pas indispensable que le procédé d'activation ait lieu après la fixation par l'anticorps exprimé selon l'invention à la surface des cellules cibles. En effet, conformément à l'invention, cet anticorps peut également se lier aux peptides tels que définis mais présents sur des cellules effectrices cytotoxiques ou de lymphocytes helper déjà activés. Par «cellules effectrices cytotoxiques» on entend désigner les macrophages, les lymphocytes T cytotoxiques (TCL) et les cellules tueuses (NK), ainsi que leurs cellules dérivées telles que par exemple les LAK, (²Versteeg, 1992, Immunology Today, 13, 244-247 ; Brittende et al., 1996, Cancer 77, 1226-1243 ; Poplack et al., 1976, Blood 48, 809-816). Par «lymphocytes T helper», on entend désigner notamment les CD4 qui permettent après activation la sécrétion de facteurs d'activation des cellules effectrices de la réponse immune (voir plus avant). Les polypeptides, et notamment les récepteurs, exprimés à la surface de ces cellules et qui sont impliqués dans l'activation de telles cellules consistent notamment en tout ou partie du complexe TCR, plus particulièrement le TCR-α, le TCR-β ou le CD3, tout ou partie des complexes CD8, CD4, CD28, LFA-1, 4-1BB (Melero et al., 1998, Eur. J. Immunol., 28, 11I6-3.121), CD47, CD2, GD1, CD9, CD45, CD30, CD40, tout ou partie des récepteurs de cytokines (Finke et al., 1998, Gene Therapy, 5, 31-39), telles que IL-7, IL-4, IL-2, IL-15 ou GM-CSF, tout ou partie du complexe récepteur des cellules NK tel que par exemple Vα14NKT (Kawano et al., 1998, Immunology 95, 5690-5693), NKAR, Nkp46 (Pessino et al., 1998, J. Exp. Med., 188, 953-960), NKp44, tout ou partie des récepteurs de macrophages tels que par exemple le récepteur Fc (Deo et al., 1997, Immunology Today, 18, 127-135). Selon un mode de réalisation particulier, il est également possible d'envisager de modifier génétiquement, notamment *in vivo*, les cellules effectrices cytotoxiques ou les lymphocytes T helper de façon à ce qu'elles expriment à leur surface un polypeptide, naturellement non exprimé par ces cellules, et capable d'induire le procédé d'activation de telles cellules, par l'introduction dans ces cellules de séquences d'acide nucléique renfermant le gène codant pour un tel polypeptide. Conformément à la présente invention, il est alors possible de sélectionner une séquence d'acide nucléique contenant un gène d'intérêt thérapeutique codant pour tout ou partie d'un anticorps susceptible d'être exprimé à la surface des cellules cibles du patient à traiter, ledit anticorps étant capable de se fixer à un tel polypeptide naturellement non exprimé par ces cellules effectrices cytotoxiques ou lymphocytes T helper.

Plus particulièrement, la présente invention repose sur la possibilité de cloner les gènes codant pour tout ou partie d'un anticorps et d'exprimer ledit anticorps dans des cellules après transfert desdits gènes dans lesdites cellules à partir des vecteurs tels que décrits précédemment. La littérature propose un grand nombre d'exemples de gènes codant pour des anticorps capables de réagir avec de tels polypeptides ou récepteurs. Il est à la portée de l'homme de l'art d'obtenir les séquences d'acides nucléiques codant pour de tels anticorps. Citons pour exemple les gènes codant pour les chaines légère et lourde de l'anticorps YTH 12.5 (anti CD3) (Routledge et al., 1991, Eur. J. Immuno. 21, 2717-2725), de l'anti CD3 selon Arakawa et al., 1996, J. Biochem., 120, 657-662). Les séquences d'acide nucléique de tels anticorps sont aisément identifiables à partir des bases de données communément utilisées par l'homme du métier.

Il est également possible, à partir d'hybridomes disponibles auprès de l'ATCC et qui sécrètent des anticorps spécifiques de polypeptides présents à la surface de cellules effectrices cytotoxiques ou de lymphocytes T helper et impliqués dans le procédé d'activation de telles cellules (par exemple un hybridome excrétant des immunoglogulines Gγ2b-κ dirigées contre les récepteurs du TCR), de cloner les séquences d'acides nucléiques codant pour les chaînes lourdes et/ou légères de ces différents anticorps par les méthodes d'amplification telles que la RT-PCR à l'aide d'oligonucléotides spécifiques ou les techniques mettant en oeuvre des banques de cADN (Maniatis et al., 1982, Molecular cloning. A laboratory manual. C.S.H. Laboratory, Cold Spring Harbor, New York). Les séquences ainsi clonées sont alors disponibles pour leur clonage dans des vecteurs. Selon un cas préféré de l'invention, la séquence d'acide nucléique codant pour la chaîne lourde de l'anticorps est fusionnée par recombinaison homologue avec la séquence d'acide nucléique codant pour un polypeptide transmembranaire tel que la glycoprotéine rabique (ces techniques de biologie moléculaires sont parfaitement décrites dans la demande de brevet français n° FR 97 09152) ou la gp160 (Polydefkis et al., 1990, J. Exp. Med., 171, 875-887).

Parmi les cellules cibles de mammifère que l'invention se propose d'éliminer ou de limiter dans leur progression, on peut citer plus spécifiquement les cellules tumorales, les cellules infectées par un agent pathogène viral ou les cellules infectées par un agent pathogène bactérien. Selon l'invention, l'expression à la surface de ces cellules de tout ou partie d'un anticorps capable de se fixer à un polypeptide présent à la surface d'une cellule effectrice cytotoxique ou d'un lymphocyte T helper et impliqué dans le procédé d'activation d'une telle cellule, permet de diriger la réponse immune cytotoxique vers une cible donnée, et plus particulièrement de diriger cette réponse au niveau d'une tumeur ou d'un foyer infectieux.

A titre d'»agent pathogène viral» on peut par exemple citer le virus VIH, EBV, CMV, le virus de l'hépatite et les papillomasvirus. Par «agent pathogène parasitaire», on peut par exemple citer Leishmania lesmaniae et Plasmodium falciparum.

Selon un mode de réalisation particulier, l'invention concerne un matériel biologique constitué par au moins une cellule cible, telle que notamment une cellule tumorale ou une cellule infectée par un agent pathogène viral, ne produisant pas naturellement des anticorps, sous une forme permettant leur administration dans l'organisme d'un mammifère, humain ou animal, ainsi qu'éventuellement leur culture préalable, ladite cellule étant génétiquement modifiée *in vitro* par au moins une séquence d'acide nucléique contenant au moins un gène codant pour tout ou partie d'un anticorps exprimé à la surface de ladite cellule cible , et ledit anticorps étant capable de se fixer à un polypeptide présent à la surface d'une cellule effectrice cytotoxique ou d'un lymphocyte T helper tel que ceux précédemment décrits. Plus particulièrement, ladite cellule cible provient soit du mammifère à traiter, soit d'un autre mammifère que celui à traiter. Dans ce dernier cas, il convient de noter que ladite cellule cible aura subi un traitement la rendant compatible avec le mammifère à traiter. Selon un cas préféré, par «mammifère» on entend désigner un mammifère humain.

Un tel matériel biologique, lorsqu'il est administré à un patient, et plus particulièrement administré par voie intratumorale, est capable d'induire chez celui-ci une réponse immunitaire à médiation cellulaire pouvant conduire à la production de cytokines et à l'effet cytotoxique des cellules effectrices qui se traduisent non seulement par l'élimination des cellules administrées mais également à l'élimination des cellules voisines présentant les antigènes, notamment tumoraux, susceptibles d'être reconnus par lesdites cellules effectrices cytotoxiques activées.

L'invention concerne par ailleurs l'utilisation d'un matériel biologique tel que décrit ci-dessus pour la préparation d'une composition pharmaceutique destinée au traitement ou à la prévention de cancers ou d'infections virales. Plus particulièrement, l'invention porte sur l'utilisation d'une séquence d'acide nucléique contenant au moins un gène d'intérêt thérapeutique et des éléments assurant l'expression dudit gène *in vivo* dans des cellules cibles génétiquement modifiées par une dite séquence d'acide nucléique, ledit gène d'intérêt thérapeutique codant pour tout ou partie d'un anticorps exprimé à la surface de ladite cellule cible et capable de se fixer à un polypeptide présent à la surface d'une cellule effectrice cytotoxique ou d'un lymphocyte T helper et impliqué dans le procédé d'activation d'une telle cellule, pour la préparation de compositions pharmaceutiques destinées à traiter un mammifère par transfert de gène.

Pour la mise en oeuvre du traitement du mammifère mentionné dans la présente invention, il est possible de disposer de compositions pharmaceutiques comprenant un matériel biologique tel que précédemment décrit, avantageusement associé avec un véhicule pharmaceutiquement acceptable pour l'administration à l'homme ou à l'animal. L'utilisation de tels supports est décrite dans la littérature. Ce véhicule pharmaceutiquement acceptable est préférentiellement isotonique, hypotonique ou présente une faible hypertonicité et a une force ionique relativement basse, tel que par exemple une solution de sucrose. Par ailleurs, ladite composition peut contenir des solvants, des véhicules aqueux ou partiellement aqueux tels que de l'eau stérile, libre d'agent pyrogène et des milieux de dispersion par exemple. Le pH de ces compositions pharmaceutiques est convenablement ajusté et tamponné selon les techniques conventionnelles.

Selon une variante, l'invention concerne une composition pharmaceutique comprenant notamment un matériel biologique comme décrit et un composé protéique naturellement responsable de ou impliqué dans l'activation des cellules effectrices cytotoxiques ou de lymphocytes T helper. Plus particulièrement, un tel composé consistera en une cytokine (The cytokine Handbook, 2eme Ed., Ed. A. W. Thomson, Ac. Press, Harcourt Brace&Company), une chemokine (Rollins et al, 1997, Blood, 90, 909-928 ; Devalaraja et al, 1999, TIPS, 20, 151-156) ou tout autre composé permettant la co-stimulation des cellules effectrices cytotoxiques (par exemple tout ou partie d'un anticorps anti-CD28 ; Stefan et al, 1997, Cancer research, 59(8), 1961-1967). De façon préférée, ledit composé sera l'IL2. Dans une variante de mise en oeuvre, le matériel biologique selon la présente invention pourra comporter, en outre, une séquence d'ADN assurant l'expression d'un composé impliqué dans l'activation des cellules effectrices cytotoxiques ou de lymphocytes T helper. Dans ce cas, cette séquence peut être contenue dans la séquence d'acide nucléique précédemment décrite ou contenue dans une séquence d'acide nucléique indépendante de celle contenant ledit gène d'intérêt thérapeutique (WO 95/09241).

Selon une première possibilité, le médicament peut être administré directement *in vivo* ou par une approche *ex vivo* qui consiste à prélever des cellules cibles au mammifère à traiter, à les transfecter *in vitro* selon l'invention et à les réadministrer audit mammifère.

Le matériel biologique selon l'invention peut être administré in vivo notamment sous forme injectable, notamment par voie intratumorale. On peut également envisager une injection par voie intratrachéale, intranasale, épidermique, intraveineuse, intra-artérielle, intramusculaire, intrapleurale, intracérébrale par seringue ou tout autre moyen équivalent. Selon un autre mode de réalisation, on peut utiliser des systèmes adaptés au traitement des voies aériennes ou des muqueuses tels que l'inhalation, l'instillation, ou l'aérosolisation, par voie topique, par administration orale ou tout autre moyen parfaitement connu de l'homme de l'art et applicable à la présente invention. L'administration peut avoir lieu en dose unique ou répétée, une ou plusieurs fois après un certain délai d'intervalle. La voie d'administration et le dosage les mieux appropriés varient en fonction de différents paramètres tels que par exemple l'individu ou la maladie à traiter, ou encore de l'acide nucléique à transférer ou de l'organe/tissus cible.

L'invention concerne également une cellule de mammifère ne produisant pas naturellement d'anticorps, caractérisée en ce qu'elle est génétiquement modifiée par au moins une séquence d'acide nucléique contenant au moins un gène d'intérêt thérapeutique et des éléments assurant l'expression dudit gène dans ladite cellule, ledit gène d'intérêt thérapeutique codant pour tout ou partie d'un anticorps exprimé à la surface de ladite cellule génétiquement modifiée et en ce que ledit anticorps est capable de se fixer à un polypeptide présent à la surface d'une cellule effectrice cytotoxique ou d'un lymphocyte T helper et impliqué dans le procédé d'activation d'une telle cellule décrit plus haut.

Enfin, l'invention concerne un procédé de préparation d'une cellule telle que décrite ci-dessus caractérisé en ce que l'on introduit dans une cellule de mammifère ne produisant pas naturellement d'anticorps, par tout moyen approprié, au moins une séquence d'acide nucléique contenant au moins un gène d'intérêt thérapeutique et des éléments assurant l'expression dudit gène dans ladite cellule, ledit gène d'intérêt thérapeutique codant pour tout ou partie d'un anticorps exprimé à la surface de ladite cellule génétiquement modifiée et en ce que ledit anticorps est capable de se fixer à un polypeptide présent à la surface d'une cellule effectrice cytotoxique ou d'un lymphocyte T helper et impliqué dans le procédé d'activation d'une telle cellule, puis en ce que l'on sélectionne parmi ces cellules celles génétiquement modifiées par ladite séquence d'acide nucléique.

Les exemples ci-après illustrent l'invention sans la limiter en aucune façon.

Légende des Figures .

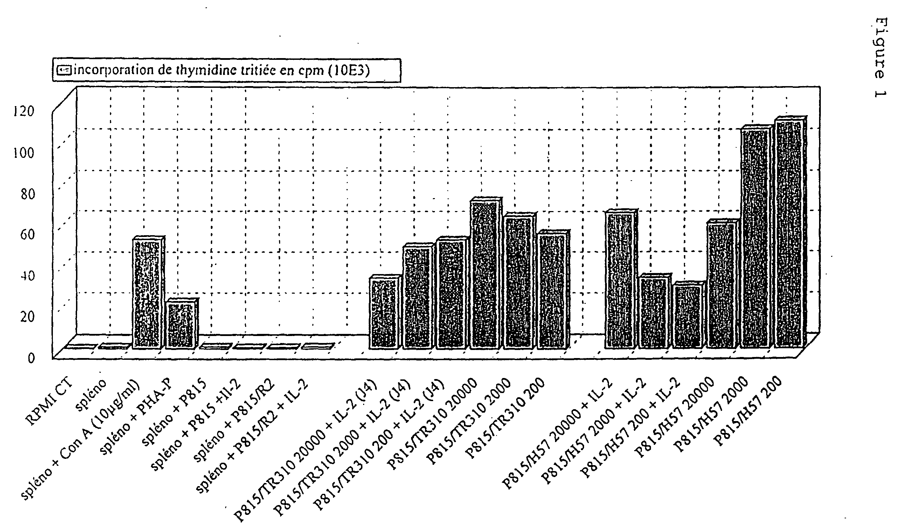

**Figure 1 :** Test de prolifération sur splénocytes murins (2E5 cellules/puits) activés avec des cellules P815 armées avec les anticorps TR310 et H57-597. Mise en présence pendant 5 jours. Marquage thymidine tritiée 8h. Les résultats sont exprimés en quantité de thymidine tritiée incorporée (10E3 cpm).

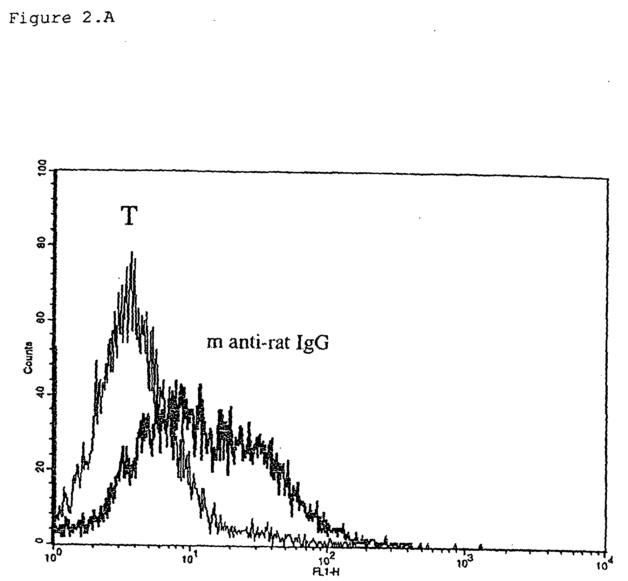

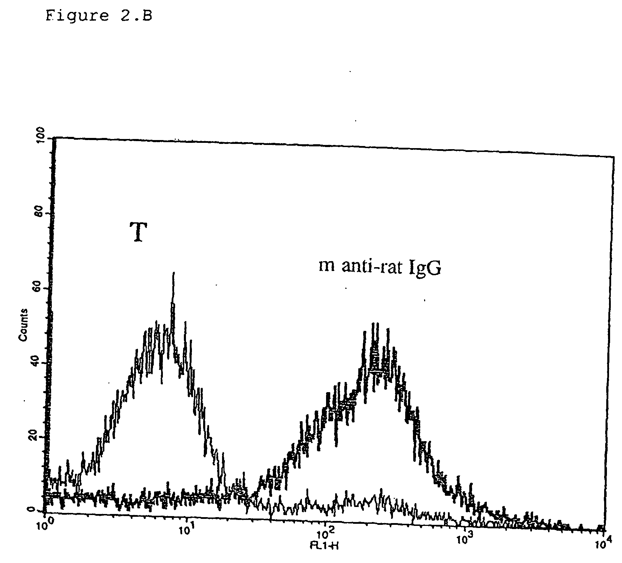

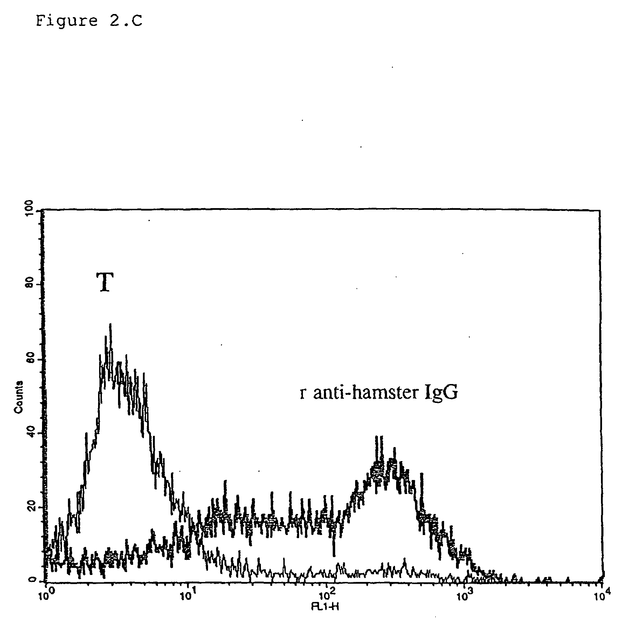

**Figure 2 :** Analyse par cytométrie de flux de l'expression des différents anticorps après infection de cellules BHK21 avec les différents virus recombinants. **A-B-C** correspondent aux infections réalisées avec les virus MVATG14205, MVATG14240 et MVATG14237, respectivement. (T : marquage négatif à l'aide d'un anticorps témoin négatif ; m anti-rat IgG : marquage à l'aide d'un anticorps de souris dirigé contre les IgG de rat ; r anti-hamster IgG : marquage à l'aide d'un anticorps de rat dirigé contre les IgG de hamster).
   Figure 2A : Analyse de l'expression de l'anticorps TR310
   Figure 2B : Analyse de l'expression de l'anticorps KT3
   Figure 2C : Analyse de l'expression de l'anticorps H57-597.

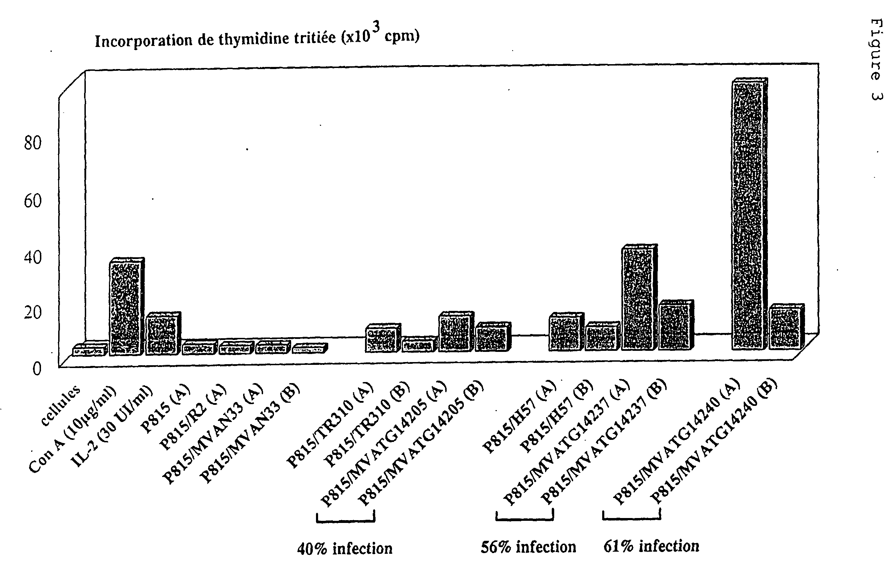

**Figure 3 : Modèle P815 (faible immunogénécité ; H2d ; CMH1 ; ICAM1** ; **CD48).** Test de prolifération sur splénocytes murins (2.10⁵ cellules/ puits) activés avec des cellules P815 infectées avec les différents virus MVA recombinants. A-B correspond à la mise en contact de 2.10⁵ splénocytes avec 20000 ou 2000 des différentes cellules P815 respectivement. P815/TR310 , P815/H57-597 et P815/R2 : P815 armées avec les anticorps TR310, H57-597 et un anticorps témoin, respectivement ; P815/MVATG14205, P815/MVATG14237, P815/MVATG14240 : P815 infectées avec les différents virus MVA recombinants ; Con A et IL-2 : témoins positifs correspondant à la stimulation de splénocytes avec de la concanavaline A ou de l'interleukine-2 recombinante ; cellules: splénocytes seuls. Le pourcentage d'infection avec les différents virus MVA recombinants est indiqué.

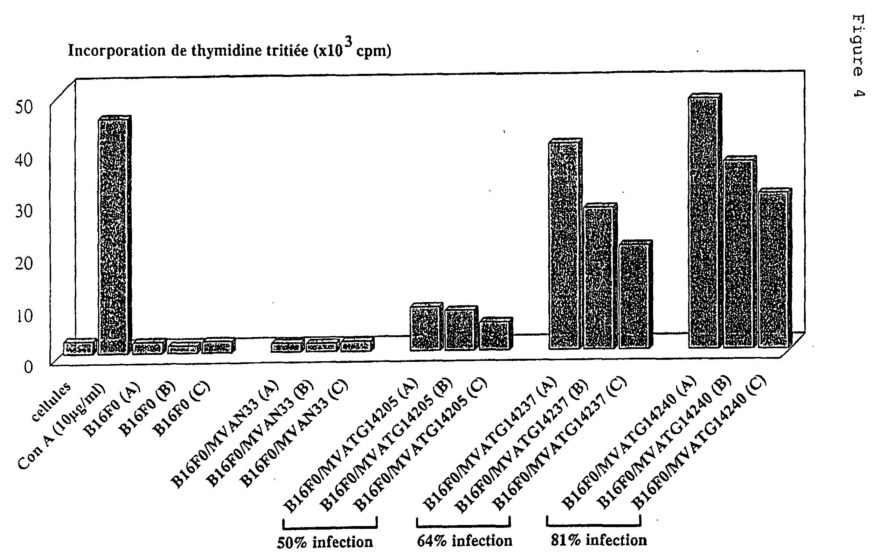

**Figure 4 : Modèle B16F0 (non immunogène ; H2b)** test de prolifération sur splénocytes murins (2.10⁵ cellules/ puits) activés avec des cellules B16F0 infectées avec les différents virus MVA recombinants. A-B-C correspond à la mise en contact de 2.10⁵ splénocytes avec 20000 , 10000 ou 2000 des différentes cellules B16F0. B16F0/MVATG14205, B16F0/MVATG14237, B16F0/MVATG14240 : B16F0 infectées avec les différents virus MVA recombinants ; Con A: témoin positif correspondant à la stimulation de splénocytes avec de la concanavaline A; cellules : splénocytes seuls. Le pourcentage d'infection avec les différents virus MVA recombinants est indiqué.

### Exemple 1

### 1 - Méthodes:

### 1-1 Construction des virus MVA recombinants

Afin de permettre le clonage des séquences d'acide nucléique codant pour différents anticorps choisis pour leur capacité à activer les lymphocytes T, trois hybridomes différents ont été sélectionnés :
- hybridome TR310 (rat anti-Vb7 murin (IgG2b); ATCC HB-219; I. L.Weissman;fusion myelomes murin/splénocytes rat).
- hybridome H57-597 (hamster anti-TCRab murin (IgG); ATCC HB-218; Kubo *et al,* 1989, J. Immunology, 142: 2736-2742; fusion myelomes murin/splénocytes hamster).
- hybridome KT3 (rat anti-CD3e murin (IgG2a); Tomonari *et al,* 1988, Immunogenetics, 28: 455-458; fusion myelomes murin/splénocytes rat).

Le clonage des séquences codant pour l'intégralité des différentes chaînes lourdes et légères de ces anticorps a été réalisé selon deux méthodes différentes à partir des ARN totaux extraits des 3 hybridomes:
a) par RT-PCR à l'aide d'oligonucléotides spécifiques ( *Bca* BEST^{TM} RNA PCRKit, Takara Shuzo Co., Ltd; Frohman *et al,* 1988, Proc. Natl. Acad. Sci. USA, 85: 8998-9002) définis de manière à ce qu'ils s'hybrident au niveau des régions conservées des séquences 3' constante et 5' variable codant pour les immunoglobulines correspondantes (voir C. A. Kettleborough et al, 1993, Eur. J. Immunol. 23: 206-211). Plus particulièrement, ces séquences ont été définies à l'aide des informations suivantes disponibles sur GeneBank :
   Hybridome TR310 et KT3 (chaînes lourdes et légères de rat) :
      - chaîne lourde de type gamma: Rat anti-acetylcholine receptor antibody gene, rearranged Ig gamma-2a chain, VDJC région, complete cds. Author: Agius, M. A. and Bharati, S. 1993. Numero d'accession dans genebank=L22654
      - chaîne légère de type kappa: Rat anti-acetylcholine receptor antibody gene, kappa-chain, VJC région, complete cds. Author: Agius, M. A. and Bharati, S. 1993. Unpublished. Numéro d'accession dans genebank=L22653.
   Hybridome H57-597: (chaîne lourde et légère de hamster):
      - chaîne lourde de type gamma: Cricetulus migratorius IgG heavy chain mRNA, complète cds. Author: Whitters, M. J. and Collins, M. 1995. Immunogenetics. 42(3): 227-228. Numéro d'accession dans genebank=U17166.
      - chaîne légère de type lambda: Mus Musculus immunoglobulin lambda chain (IgL) mRNA, complete cds. Author: Reidl, L. S. Kinoshita, C. M. and Steiner, L. A. 1992. J. Immunol.149: 471-480. Numero d'accession dans genebank=M94349.
b) par constructions de banques de cDNA en pBluescript ( Universal Riboclone System, Promega, Madison, USA: Maniatis *et al*, 1982, Molecular cloning. A laboratory manual. C. S. H. Laboratory, Cold Spring Harbor,New York) et screening de ces banques avec les fragments amplifiés selon a).

Les chaînes ainsi isolées sont sous-clonées par recombinaison dans un virus MVA recombinant renfermant la séquence d'acide nucléique codant pour la région transmembranaire du virus de la rage (Modified Vaccinia Ankara; Antoine et al, 1998, Virology, 244: 365-396 et demande de brevet français n° FR 97 09152) afin d'obtenir des virus recombinants capables d'exprimer des anticorps fonctionnels capables de reconnaître et de se lier à leur antigène spécifique et exprimés de manière transmembranaire à la surface des cellules recombinées.

### 1-2 Etude in vitro de l'effet des anticorps TR310 et H57-597 sur la prolifération de splénocytes murins.

Des splénocytes murins (souche DBA/2) ont été stimulés avec des cellules tumorales murines P815HT (souche C57/816; Acres *et al,* 1993, J. Immunother. 14: 136-143) en présence de l'un ou l'autre des deux anticorps (TR310 et H57-597) purifiés à partir des surnageants d'hybridome correspondant, pendant 1h à 37°C. La présence à la surface des cellules P815HT de récepteurs à immunoglobulines de type Fc permet le recouvrement desdites cellules avec les anticorps. Après 5 jours d'incubation entre les cellules P815 présentant à leur surface les anticorps sélectionnés et les splénocytes, la prolifération des lymphocytes T est mesurée par un test d'incorporation de thymidine tritiée (Gimmi et al, 1996, Nat. Med. 12: 1367-1371).

Par ailleurs, lors de cette étude, plusieurs paramètres ont été analysés:
a) nombre de cellules P815 présentant à leur surface les anticorps sélectionnés nécessaire à la stimulation ;
b) co-stimulation éventuelle à l'aide d'IL-2 recombinante humaine au jour 4 (100 ng/ml soit 50 UI/ml; R&Dsystems)

### 1-3 Etude de la réponse Th (T helper) associée à la stimulation des splénocytes par les différents anticorps (TR310 et H57-597) présentés à la surface des cellules P815.

Pour évaluer cette réponse, les surnageants du test de prolifération précédent, réalisé en présence d'IL-2 recombinante humaine, ont été prélevés au jour 4 ou 5. La présence dans ces surnageants d'IL-4 (réponse de type Th2) ou d'IFNγ (réponse de type Th1) a été mesuré à l'aide de kits spécifiques disponibles dans le commerce, selon les recommandations du fournisseur (Kit Quantikine, R&D systems).

### 2 - Résultats

### 2-1 Etude in vitro de l'effet des anticorps TR310 et H57-597 sur la prolifération de splénocytes murins.

### 2 - 1 - 1 - Contrôles

Parallèlement à cette étude, un certain nombre de témoins ont également été analysés.

Il s'agit plus particulièrement de mesurer les effets observés dans des conditions identiques au test proprement dit de :
RPMI CT: témoin négatif renfermant le milieu seul.
Spléno: témoin négatif renfermant les cellules splénocytes seules.
Spléno + P815 +/- IL-2: témoin négatif renfermant les cellules splénocytes + les cellules P815 cibles non armées, en présence ou en absence (+/-) d'IL-2 (100 ng/ml) ajoutée au jour 4.
Spléno + P815/R2 +/- IL-2: témoin négatif renfermant les cellules splénocytes + les cellules P815 cibles portant à leur surface un anticorps neutre (non capable d'induire l'activation des lymphocytes) +/- IL-2 (100 ng/ml) ajoutée au jour 4.
Spléno + ConA: témoin positif correspondant à la stimulation des splénocytes murins à l'aide de la Concanavaline A (10 µg/ml) qui est un mitogène murin puissant.
Spléno + PHA-P: témoin positif correspondant à la stimulation des splénocytes murins à l'aide de la phytohématogluttinine A (1 µg/ml) qui est un mitogène humain puissant.

### 2 - 1 - 2 - Test

P815/TR310 (20000/2000/200)+/-IL-2: Evaluation de la stimulation de splénocytes murins par des cellules P815 cibles portant à leur surface un anticorps TR310 (2E4, 2E3, 2E2 cellules/test) en présence ou non d'IL-2 recombinante humaine à partir de J+4.

P815/H57-597 (20000/2000/200)+/-IL-2: Evaluation de la stimulation de splénocytes murins par des cellules P815 cibles portant à leur surface un anticorps H57-597 (2E4, 2E3, 2E2 cellules/test) en présence ou non d'IL-2 recombinante humaine à partir de J+4.

Les résultats de stimulation obtenus sont présentés sur la Figure 1. Ces résultats montrent que la présentation d'anticorps «activateurs» de lymphocytes T à la surface des cellules cibles P815 permet l'activation et la prolifération de splénocytes murins (visualisées par l'incorporation de thymidine tritiée). L'ajout d'interleukine-2 recombinante humaine ne semble pas amplifier cette stimulation anticorps-dépendante. Par ailleurs, nous avons également montré que l'activation des lymphocytes telle que mesurée s'accompagne de la lyse des cellules cibles P815.

### 2-2 Etude de la réponse Th (T helper) associée à la stimulation des splénocytes par les différents anticorps (TR310 et H57-597).

Les résultats observés sont présentés sur le Tableau 1 suivant qui indique les mesures des dosages de l'IL-4 et de l'IFNγ après activation de splénocytes murins par les anticorps TR310 et H57-597 présents à La surface de cellules tumorales murines P815.

| cellules activatrices | P815 2000 cellules/test | P815-TR310 2000 cellules/test | P815-H57-597 2000 cellules/test |
|---|---|---|---|
| dosage de l'IL-4 produite en pg/ml | 0 | 31 | 23.5 |
| dosage de l'IFNg produite en pg/ml | 0 | 92.9 | 162.75 |

Ces dosages montrent que les anticorps TR310 et H57-597 dans un contexte de présentation membranaire induisent les 2 types de réponse Th1 et Th2 (tableau 1). Les anticorps TR310 et H57-597 sont donc capables lorsqu'ils sont présentés à la surface de cellules tumorales d'activer une réponse de type «helper» Th1 mais aussi Th2 en se liant à leur récepteur membranaire présent à la surface des cellules T CD4+. La sécrétion de ces cytokines permet en particulier d'augmenter la réponse immunitaire dirigée contre les cellules tumorales.

### Exemple 2

### 1- Méthodes :

### 1-1 Construction des virus MVA recombinants

Trois virus MVA recombinants ont été construits (MVATG14205 exprimant l'anticorps de rat TR310 (anti-V beta 7 murin) ; MVATG14237 exprimant l'anticorps de hamster H57-597 (anti-TCR alpha/beta de hamster) et MVATG14240 exprimant l'anticorps de rat KT3 (anti-CD3 epsilon de rat). Les cassettes d'expression ont été introduites dans la délétion II du MVA (Modified Virus Ankara) comme décrit dans la demande de brevet WO 9903885. La chaîne légère de chaque anticorps a été placée sous contrôle du promoteur vaccine early-late p7.5 (Goebel et al, 1990, Virology, 179, 247-266). La séquence codant pour la chaîne lourde de chaque anticorps a été placée sous le contrôle du promoteur vaccine early-late pH5R (Goebel et al, 1990, Virology, 179, 247-266). Afin de faciliter la sélection des virus MVA recombinants, le gène de sélection GPT (xanthine-guanine phosphoribosyltransférase) de E. coli est utilisé. L'extrémité C-terminale de la chaîne lourde de chaque anticorps est en outre fusionnée avec le domaine trans-membranaire et intra-cytoplasmique de la glycoprotéine rabique afin de permettre l'ancrage des anticorps dans la membrane plasmique.

### 1-2 Evaluation de l'expression des différents anticorps après infection de cellules embryonnaires de poulet (CEP) avec les différents virus MVA recombinants.

L'expression est analysée par Western Blot en respectant les conditions du kit «ECL^{TM} Western Blotting » d'Amersham Life Science (UK). Pour cela, des cellules embryonnaires de poulet sont infectées à une multiplicité d'infection (MOI) de 1 avec les différents virus MVA recombinants. Les mêmes cellules sont également infectées avec le virus contrôle MVAN33. Après 24h d'infection, les cellules sont lavées avec du PBS puis soumises à sonication dans le tampon de dépôt. La suspension est ensuite dénaturée 3 min à 95°C avant d'être fractionnée sur un gel de polyacrylamide 13%. Les protéines ainsi fractionnées sont ensuite transférées sur une membrane PVDF (porablot ; Macherey-Nagel). L'expression des anticorps TR310 et KT3 est analysée après hybridation à l'aide d'un anticorps de souris dirigé contre les IgG de rat couplé à la HRPO (horseradish peroxidase ; 10 µg/ml). L'expression de l'anticorps H57-597 est analysée après hybridation à l'aide d'un anticorps de rat dirigé contre les IgG de hamster couplé à la HRPO.

### 1-3 Analyse par cytométrie de flux de l'expression des différents anticorps après infection de cellules BHK21 avec les différents virus recombinants.

L'expression est analysée par FACS après infection de cellules BHK21 (Baby Hamster Kidney) à une MOI de 1 avec les différents virus MVA recombinants. Après 12 h d'infection , les cellules sont décollées puis analysées. La détection des anticorps TR310 et KT3 est réalisée après incubation avec un anticorps de souris dirigé contre les IgG de rat, couplé à la FITC (Jackson ImmunoResearch Laboratories Inc (Pennsylvania ; USA)). La détection de l'anticorps H57-597 est réalisée à l'aide d'un anticorps de rat dirigé contre les IgG de hamster couplé à la FITC (Jackson ImmunoResearch Laboratories Inc (Pennsylvania ; USA)). Les cellules infectées ont également été incubées avec un anticorps témoin.

### 1-4 Etude in vitro de la fonctionnalité des différents virus MVA recombinants.

La fonctionnalité des virus MVA recombinants est testée en réalisant des test de prolifération sur splénocytes murins. Des splénocytes murins (souche DBA/2) sont stimulés avec des cellules tumorales murines P815HT ou B16F0 infectées avec les 3 virus MVA recombinants. Pour cela, des cellules P815HT et B16F0 sont infectées à une MOI de 1 pendant 20h avec les virus MVATG14205, MVATG14237, MVATG14240 et MVAN33 (contrôle négatif). Pour le modèle P815HT, les cellules sont également mises en contact avec 10 µg/ml des anticorps TR310 et H57-597 purifiés à partir de surnageant d'hybridome. Les cellules infectées ou armées sont ensuite traitées pendant 1 h avec de la mitomycine C (Sigma ; 50 µg/ml) afin de stopper leur division. Après 5 jours d'incubation dans les différentes conditions de cellules P815 et B16F0 et les splénocytes murins, la prolifération des lymphocytes T est mesurée par un test d'incorporation de thymidine tritiée.

### 2- Résultats :

### 2-1 Construction des MVA recombinants

Les virus MVA recombinants (MVATG14205; MVATG14237 et MVATG14240) sont générés par recombinaison homologue dans des cellules embryonnaires de poulet à l'aide des différents plasmides de transfert (pTG14205; pTG14237 et pTg14240) et d'un virus MVA sauvage MVAN33 comme décrit dans la demande de brevet WO 9903885.

### 2-2 Evaluation de l'expression des différents anticorps après infection de cellules embryonnaires de poulet (CEP) avec les différents virus MVA recombinants.

L'expression des différents anticorps (TR310, H57-597 et KT3) est analysée par Western Blot après infection de CEP avec les virus MVA recombinants. Les résultats observés montrent qu'il y a bien expression d'immunoglobuline de type IgG de rat dans les cellules infectées avec les virus MVATG14205 et MVATG14240. Après infection avec le MVATG14237, il y a également expression d'une immunoglobuline de type IgG de hamster. Ces résultats sont révélés par la présence d'une bande spécifique migrant à environ 60 kDa et correspondant à la chaîne lourde de l'immunoglobuline. Aucune bande spécifique n'a pu être observée après infection de CEP par le virus contrôle MVAN33.

### 2-3 Analyse par cytométrie de flux de l'expression des différents anticorps après infection de cellules BHK21 avec les différents virus recombinants.

L'expression et l'assemblage des 3 anticorps sont évalués par cytométrie de flux (Figure 2). Des cellules BHK21 sont infectées à une MOI de 1 avec les 3 virus recombinants. L'analyse par FACS a permis de mettre en évidence l'expression d'IgG de surface de hamster (H57-597) et de rat (TR310 et KT3) après incubation des cellules infectées avec des anticorps dirigés spécifiquement contre des IgG de rat et de hamster. Cette analyse a également permis de montrer l'expression de chaîne légère de type kappa dans les cellules BHK21 infectées avec les virus MVATG14205 et MVATG14240. Ces résultats permettent de montrer que l'infection de cellules BHK21 par les différents virus MVA recombinants entraîne l'expression d'immunoglobulines membranaires correctement réarrangées.

### 2-4 Etude in vitro de la fonctionnalité des différents virus MVA recombinants.

La fonctionnalité des virus MVA recombinants est testée en réalisant des test de prolifération sur splénocytes murins. Pour cela 2 types de modèles murins sont utilisés, les cellules P815HT (Figure 3) et les cellules B16F0 (Figure 4). Ces 2 types de cellules sont infectés avec les virus recombinants afin d'exprimer à leur surface des anticorps transmembranaires dirigés contre tout ou partie du complexe TCR/CD3 murin. Nous avons mesuré la prolifération induite par la mise en contact de cellules P815HT et B16F0 infectées avec des splénocytes murins naifs d'un haplotype semblable. Dans un premier temps, des cellules P815HT (faiblement immunogène ; H2d ; exprimant le CMHI, ICAMI et CD48) sont infectées avec les 3 virus recombinants et un virus MVAN33 contrôle. En parallèle les mêmes cellules P815HT sont incubées avec les anticorps TR310 et H57-597 afin de les armer d'anticorps par l'intermédiaire de leur récepteur Fc. Pour le virus MVATG14205 (stimulation des cellules Vbéta 7 murines), la stimulation induite par des cellules P815 infectées est faiblement supérieure à celle obtenue avec les cellules armées. De plus, le niveau de prolifération reste très faible comme attendu avec la spécificité de l'anticorps. Avec les virus MVATG14237 et MVATG14240, l'indice obtenu avec les cellules infectées est nettement supérieur à celui obtenu avec les cellules armées. Les indices obtenus avec les cellules infectées par les 3 virus sont très nettement supérieurs à ceux obtenus avec des agents mitogènes puissants comme la concanavaline A ou l'interleukine-2. Enfin, nous avons pu mettre en évidence dans tous les cas un effet dose proportionnel au nombre de cellules infectées mis en contact avec les splénocytes. Aucune stimulation n'a pu être observée avec des cellules P815HT non infectées, armées avec un anticorps témoins ou infectées avec un virus contrôle.

De manière identique, les cellules B16F0 sont utilisées dans le même type d'expérience (Figure 4). Ces cellules murines B16F0 sont non immunogènes et ne possèdent aucune molécule de co-stimulation. Nous avons ainsi mis en évidence une forte prolifération de splénocytes naïfs après contact avec des cellules infectées. De façon identique, les cellules infectées avec le MVATG14205 induisent une prolifération inférieure aux cellules infectées avec les virus MVATG14237 et MVATG14240. Pour ces deux derniers, l'indice de prolifération obtenu avec 20000 cellules est comparable à celui obtenu avec la Con A. Dans ce modèle, la quantité de cellules infectées semble également jouer sur l'indice de prolifération obtenu (effet dose).

En conclusion, il apparaît très clairement que les 3 virus recombinants sont fonctionnels. L'expression des anticorps TR310, H57-597 et KT3 à la surface de cellules permet d'induire, selon l'invention, une forte prolifération de cellules T naïves. Cette stimulation est de manière avantageuse plus importante que celle obtenue avec des cellules armées avec les mêmes anticorps par l'intermédiaire de récepteur de type Fc, mais également que celle obtenue avec de puissants agents mitogènes tels que la ConA.

## Revendications

1. Matériel biologique pour la préparation de compositions pharmaceutiques destinées au traitement de mammifères comprenant au moins une séquence d'acide nucléique comprenant :
(i) un gène codant pour un anticorps ou fragment d'anticorps fusionné avec le domaine transmembranaire d'un polypeptide transmembranaire, et
(ii) des éléments assurant l'expression dudit gène in vivo dans des cellules cibles destinées à être génétiquement modifiées par ladite séquence d'acide nucléique ;
**caractérisé en ce que** ledit anticorps ou fragment d'anticorps est exprimé à là surface de la cellule cible et est capable de se fixer à un récepteur sélectionné parmi le groupe consistant au récepteur du complexe TCR, plus particulièrement le TCR-α, le TCR-β ou le CD3, le CD8, le CD4, CD28, LFA-1, 4-1BB, CD47, CD2, CD9, CD45, CD40, aux récepteurs de cytokines, telles que IL-7, IL-4, IL-2, IL-15 ou GM-CSF, au Vα14NKT, NKAR, au récepteur Fc.

2. Matériel biologique pour la préparation de compositions pharmaceutiques destinées au traitement de mammifères comprenant au moins. une cellule cible ne produisant pas naturellement des anticorps **caractérisé en ce que** ladite cellule cible est modifiée in vitro par au moins une séquence d'acide nucléique telle que définie dans la revendication 1.

3. Matériel biologique selon la revendication 1 ou 2 **caractérisé en ce que** ladite séquence d'acide nucléique est sous la forme d'une séquence d'ADN ou d'ARN nue.

4. Matériel biologique selon la revendication 1 ou 2 **caractérisé en ce que** ladite séquence d'acide nucléique est un vecteur permettant le transfert dudit gène dans lesdites cellules cibles.

5. Matériel biologique selon la revendication 4 **caractérisé en ce que** ledit vecteur est un vecteur viral.

6. Matériel biologique selon la revendication 5 **caractérisé en ce que** ledit vecteur viral est un vecteur adénoviral, rétroviral, un poxvirus, notamment dérivé du virus de la vaccine ou du Modifed Virus Ankara (MVA).

7. Matériel biologique selon la revendication 4 **caractérisé en ce que** ledit vecteur consiste en au moins une dite séquence d'acide nucléique complexée ou conjuguée à au moins une molécule ou substance porteuse sélectionnée parmi le groupe consistant en un amphiphile cationique, notamment un lipide cationique, un polymère cationique ou neutre, un composé polaire protique notamment choisi parmi le propylène glycol, le polyéthylène glycol, le glycérol, l'éthahol, la 1-méthyl L-2-pyrrolidone ou leurs dérivés, et un composé polaire aprotique notamment choisi parmi le diméthylsulfoxide (DMSO), le diéthylsulfoxide, le di-n-propylsulfoxid'e, le diméthylsulfone, le sulfolane, la diméthylformamide, le diméthylacétamide, la tétraméthylurée, l'acétonitrile ou leurs dérivés.

8. Matériel biologique selon l'une des revendications 1 à 7 **caractérisé en ce que** ledit polypeptide transmembranaire est sélectionné parmi le groupe consistant en une glycoprotéine, une lipoprotéine, un récepteur membranaire.

9. Matériel biologique selon la revendication 8 **caractérisé en ce que** ledit polypeptide transmembranaire est sélectionné parmi le groupe consistant en la glycoprotéine du virus de la rage, la gp160, le CD4.

10. Matériel biologique selon l'une quelconque des revendications précédentes **caractérisé en ce que** ladite cellule cible est une cellule de mammifère tumorale, une cellule de mammifère infectée par un agent pathogène viral ou une cellule de mammifère infectée par un agent pathogène bactérien.

11. Matériel biologique selon la revendication 2 **caractérisé en ce qu'**il est sous une forme permettant leur administration dans l'organisme d'un mammifère ainsi qu'éventuellement leur culture préalable

12. Matériel biologique selon la revendication 11 **caractérisé en ce que** lesdites cellules cibles proviennent du mammifère à traiter.

13. Matériel biologique selon la revendication 11 **caractérisé en ce que** lesdites cellules cibles proviennent d'un autre mammifère que celui à traiter et ont subi un traitement les rendant compatibles.

14. Matériel biologique selon l'une des revendications 1 à 13, **caractérisé en ce qu'**il comporte, en outre, au moins une séquence d'ADN assurant l'expression d'un composé impliqué dans l'activation des cellules effectrices cytotoxiques ou de lymphocytes T helper.

15. Utilisation d'un matériel biologique selon l'une des revendications 1 à 13 pour la préparation d'une composition pharmaceutique destinée au traitement ou à la prévention de cancers ou d'infections virales.

16. Utilisation d'une séquence d'acide nucléique comprenant :
(i) un gène codant pour un anticorps ou un fragment d'anticorps fusionné avec le domaine transmembranaire d'un polypeptide transmembranaire, et
(ii) des éléments assurant l'expression dudit gène in vivo dans des cellules cibles destinées à être génétiquement modifiées par ladite séquence d'acide nucléique;
**caractérisé en ce que** ledit anticorps ou fragment d'anticorps est capable de se fixer à un récepteur qui est sélectionné parmi le groupe consistant à tout ou partie du complexe TCR pour la préparation de compositions pharmaceutiques destinées à traiter un mammifère par transfert de gène.

17. Composition pharmaceutique comprenant un matériel biologique selon l'une des revendications 1 à 14, avantageusement en association avec un véhicule pharmaceutiquement acceptable.

18. Composition. pharmaceutique selon la revendication 17, comprenant un matériel biologique selon l'une des revendications 11 à 13 et un composé naturellement responsable de l'activation des cellules effectrices cytotoxiques ou de lymphocytes T helper.

19. Composition pharmaceutique selon la revendication 18, **caractérisée en ce que** ledit composé est une cytokine ou une chemokine.

20. Cellule de mammifère non germinale ne produisant pas naturellement d'anticorps, **caractérisée en ce qu'**elle est génétiquement modifiée par au moins une séquence d'acide nucléique comprenant :
(i) un gène codant pour un anticorps ou un fragment d'anticorps fusionné avec le domaine transmembranaire d'un polypeptide transmembranaire, et
(ii) des éléments assurant l'expression dudit gène in vivo dans des cellules cibles destinées à être génétiquement modifiées par ladite séquence d'acide nucléique ;
**caractérisé en ce que** ledit anticorps ou fragment d'anticorps est capable de se fixer à un récepteur qui est sélectionné parmi le groupe consistant à tout ou partie du complexe TCR.

21. Procédé de préparation in vitro d'une cellule selon la revendication 20, **caractérisé en ce que** l'on introduit dans une cellule de mammifère non germinale ne produisant pas naturellement d'anticorps, par tout moyen approprié, au moins une séquence d'acide nucléique comprenant :
(i) un gène codant pour un anticorps ou un fragment d'anticorps fusionné avec le domaine transmembranaire d'un polypeptide transmembranaire, et
(ii) des éléments assurant l'expression dudit gène in vivo dans des cellules cibles destinées à être génétiquement modifiées par ladite séquence d'acide nucléique ;
**caractérisé en ce que** ledit anticorps ou fragment d'anticorps est capable de se fixer à un récepteur qui est sélectionné parmi le groupe consistant à tout ou partie du complexe TCR, puis **en ce que** l'on sélectionne parmi ces cellules celles génétiquement modifiées par ladite séquence d'acide nucléique.

## Patentansprüche

1. Biologisches Material für die Herstellung von pharmazeutischen Zusammensetzungen, welche für die Behandlung von Säugetieren bestimmt sind, umfassend wenigstens eine Nukleinsäuresequenz, umfassend:
(i) ein Gen, welches einen Antikörper oder ein Antikörperfragment fusioniert mit der Transmembrandomäne eines Transmembranpolypeptids kodiert, und
(ii) Elemente, welche die Expression des Gens in vivo in Zielzellen, welche dazu bestimmt sind, durch die genannte Nukleinsäuresequenz genetisch modifiziert zu werden, sicherstellen;
**dadurch gekennzeichnet, dass** der Antikörper oder das Antikörperfragment auf der Oberfläche der Zielzelle exprimiert wird und in der Lage ist, an einen Rezeptor, ausgewählt aus der Gruppe bestehend aus dem Rezeptor des TCR-Komplexes, insbesondere TCR-a, TCR-b oder CD3, CD8, CD4, CD28, LFA-1, 4-1 BB, CD47, CD2, CD9 CD45, CD40, den Rezeptoren von Zytokinen, wie IL-7, IL-4, IL-2, IL-15 oder GM-CSF, dem Va14NKT, NKAR, dem Fc-Rezeptor, zu binden.

2. Biologisches Material für die Herstellung von pharmazeutischen Zusammensetzungen, welche für die Behandlung von Säugetieren bestimmt sind, umfassend wenigstens eine Zielzelle, welche von Natur aus keine Antikörper produziert, **dadurch gekennzeichnet, dass** die Zielzelle in vitro durch wenigstens eine Nukleinsäuresequenz, wie sie in Anspruch 1 definiert wird, modifiziert ist.

3. Biologisches Material nach Anspruch 1 oder 2, **dadurch gekennzeichnet, dass** die Nukleinsäuresequenz in Form einer nackten DNA- oder RNA-Sequenz vorliegt.

4. Biologisches Material nach Anspruch 1 oder 2, **dadurch gekennzeichnet, dass** die Nukleinsäuresequenz ein Vektor ist, welcher den Transfer des Gens in die Zielzellen erlaubt.

5. Biologisches Material nach Anspruch 4, **dadurch gekennzeichnet, dass** der Vektor ein viraler Vektor ist.

6. Biologisches Material nach Anspruch 5, **dadurch gekennzeichnet, dass** der virale Vektor ein adenoviraler Vektor, retroviraler Vektor, ein Poxvirus, insbesondere abgeleitet von dem Vacciniavirus oder dem Modified Virus Ankara (MVA), ist.

7. Biologisches Material nach Anspruch 4, **dadurch gekennzeichnet, dass** der Vektor aus wenigstens einer genannten Nukleinsäuresequenz besteht, welche komplexiert oder konjugiert ist mit wenigstens einem Trägermolekül oder einer Trägersubstanz, ausgewählt aus der Gruppe bestehend aus einer amphiphilen kationischen Substanz, insbesondere einem kationischen Lipid, einem kationischen oder neutralen Polymer, einer protischen polaren Verbindung, welche insbesondere unter Propylenglycol, Polyethylenglycol, Glycerol, Ethanol, 1-Methyl-L-2-pyrrolidon oder deren Derivaten ausgewählt wird, und einer aprotischen polaren Verbindung, welche insbesondere unter Dimethylsulfoxid (DMSO), Diethylsulfoxid, Di-n-propylsulfoxid, Dimethylsulfon, Sulfolan, Dimethylformamid, Dimethylacetamid, Tetramethylharnstoff, Acetonitril oder deren Derivaten ausgewählt wird.

8. Biologisches Material nach einem der Ansprüche 1 bis 7, **dadurch gekennzeichnet, dass** das Transmembranpolypeptid aus der Gruppe bestehend aus einem Glycoprotein, einem Lipoprotein, einem Membranrezeptor ausgewählt wird.

9. Biologisches Material nach Anspruch 8, **dadurch gekennzeichnet, dass** das Transmembranpolypeptid aus der Gruppe bestehend aus dem Glycoprotein des Tollwutvirus, gp160 und CD4 ausgewählt wird.

10. Biologisches Material nach einem der vorangegangenen Ansprüche, **dadurch gekennzeichnet, dass** die Zielzelle eine Säugetier-Tumorzelle, eine durch ein virales pathogenes Agens infizierte Säugetierzelle oder eine durch ein bakterielles pathogenes Agens infizierte Säugetierzelle ist.

11. Biologisches Material nach Anspruch 2, **dadurch gekennzeichnet, dass** es in einer Form vorliegt, welche dessen Verabreichung in den Organismus eines Säugetiers wie auch gegebenenfalls dessen vorab erfolgende Kultivierung erlaubt.

12. Biologisches Material nach Anspruch 11, **dadurch gekennzeichnet, dass** die Zielzellen von dem zu behandelnden Säugetier stammen.

13. Biologisches Material nach Anspruch 11, **dadurch gekennzeichnet, dass** die Zielzellen von einem anderen Säugetier als jenem, welches zu behandeln ist, stammen und eine Behandlung, welche diese kompatibel oder verträglich macht, durchlaufen haben.

14. Biologisches Material nach einem der Ansprüche 1 bis 13, **dadurch gekennzeichnet, dass** es außerdem wenigstens eine DNA-Sequenz umfasst, welche die Expression einer Verbindung, welche an der Aktivierung der zytotoxischen Effektorzellen oder von T-Helfer-Lymphozyten beteiligt ist, sicherstellt.

15. Verwendung eines biologischen Materials nach einem der Ansprüche 1 bis 13 für die Herstellung einer pharmazeutischen Zusammensetzung, welche für die Behandlung oder die Verhütung von Krebserkrankungen oder von viralen Infektionen bestimmt ist.

16. Verwendung einer Nukleinsäuresequenz, umfassend:
(i) ein Gen, welches einen Antikörper oder ein Antikörperfragment fusioniert mit der Transmembrandomäne eines Transmembranpolypeptids kodiert, und
(ii) Elemente, welche die Expression des Gens in vivo in Zielzellen, welche dazu bestimmt sind, durch die genannte Nukleinsäuresequenz genetisch modifiziert zu werden, sicherstellen;
**dadurch gekennzeichnet, dass** der Antikörper oder das Antikörperfragment in der Lage ist, an einen Rezeptor, der ausgewählt wird aus der Gruppe bestehend aus der Gesamtheit oder einem Teil des TCR-Komplexes, zu binden, für die Herstellung von pharmazeutischen Zusammensetzungen, welche für die Behandlung eines Säugetiers durch Gentransfer bestimmt sind.

17. Pharmazeutische Zusammensetzung, welche ein biologisches Material nach einem der Ansprüche 1 bis 14, vorteilhafterweise in Kombination mit einem pharmazeutisch annehmbaren Träger oder Vehikel umfasst.

18. Pharmazeutische Zusammensetzung nach Anspruch 17, welche ein biologisches Material nach einem der Ansprüche 11 bis 13 und eine Verbindung, welche von Natur aus für die Aktivierung der zytotoxischen Effektorzellen oder von T-Helfer-Lymphozyten verantwortlich ist, umfasst.

19. Pharmazeutische Zusammensetzung nach Anspruch 18, **dadurch gekennzeichnet, dass** die Verbindung ein Zytokin oder ein Chemokin ist.

20. Nicht-germinale Säugetierzelle, welche von Natur aus keine Antikörper produziert, **dadurch gekennzeichnet, dass** sie genetisch modifiziert ist durch wenigstens eine Nukleinsäuresequenz, umfassend:
(i) ein Gen, welches einen Antikörper oder ein Antikörperfragment fusioniert mit der Transmembrandomäne eines Transmembranpolypeptids kodiert, und
(ii) Elemente, welche die Expression des Gens in vivo in Zielzellen, welche dazu bestimmt sind, durch die genannte Nukleinsäuresequenz genetisch modifiziert zu werden, sicherstellen;
**dadurch gekennzeichnet, dass** der Antikörper oder das Antikörperfragment in der Lage ist, an einen Rezeptor, der ausgewählt wird aus der Gruppe bestehend aus der Gesamtheit oder einem Teil des TCR-Komplexes, zu binden.

21. Verfahren zur in vitro-Herstellung einer Zelle nach Anspruch 20, **dadurch gekennzeichnet, dass** man in eine nicht-germinale Säugetierzelle, welche von Natur aus keine Antikörper produziert, durch ein jegliches geeignetes Mittel oder eine jegliche geeignete Maßnahme wenigstens eine Nukleinsäuresequenz einschleust, umfassend:
(i) ein Gen, welches einen Antikörper oder ein Antikörperfragment fusioniert mit der Transmembrandomäne eines Transmembranpolypeptids kodiert, und
(ii) Elemente, welche die Expression des Gens in vivo in Zielzellen, welche dazu bestimmt sind, durch die genannte Nukleinsäuresequenz genetisch modifiziert zu werden, sicherstellen;
**dadurch gekennzeichnet, dass** der Antikörper oder das Antikörperfragment in der Lage ist, an einen Rezeptor, der ausgewählt wird aus der Gruppe bestehend aus der Gesamtheit oder einem Teil des TCR-Komplexes zu binden, dann dadurch, dass man unter diesen Zellen jene, die durch die genannte Nukleinsäuresequenz genetisch modifiziert worden sind, selektiert.

## Claims

1. Biological material for preparing pharmaceutical compositions intended for treating mammals, comprising:
(i) a gene encoding an antibody or antibody fragment fused with the transmembrane domain of a transmembrane polypeptide, and
(ii) elements providing the expression of said gene in vivo in target cells intended to be genetically modified by said nucleic acid sequence;
**characterized in that** said antibody or antibody fragment is expressed at the surface of the target cell and is capable of binding to a receptor selected from the group consisting of the TCR complex receptor, more particularly TCR-α, TCR-β or CD3, CD8, CD4, CD28, LFA-1, 4-1BB, CD47, CD2, CD9, CD45 or CD40, of the receptors for cytokines, such as IL-7, IL-4, IL-2, IL-15 or GM-CSF, of Vα14NKT and NKAR and of the Fc receptor.

2. Biological material for preparing pharmaceutical compositions intended for treating mammals, comprising at least one target cell which does not naturally produce antibodies, **characterized in that** said target cell is modified in vitro with at least one nucleic acid sequence as defined in Claim 1.

3. Biological material according to Claim 1 or 2, **characterized in that** said nucleic acid sequence is in the form of a naked DNA or RNA sequence.

4. Biological material according to Claim 1 or 2, **characterized in that** said nucleic acid sequence is a vector which allows the transfer of said gene into said target cells.

5. Biological material according to Claim 4, **characterized in that** said vector is a viral vector.

6. Biological material according to Claim 5, **characterized in that** said viral vector is an adenoviral or retroviral vector, or a poxvirus, in particular derived from the vaccinia virus or from the Modified Virus Ankara (MVA).

7. Biological material according to Claim 4, **characterized in that** said vector consists of at least one said nucleic acid sequence complexed with or conjugated to at least one carrier molecule or substance selected from the group consisting of a cationic amphiphile, in particular a cationic lipid, a cationic or neutral polymer, a protic polar compound in particular chosen from propylene glycol, polyethylene glycol, glycerol, ethanol and 1-methyl-L-2-pyrrolidone or their derivatives, and an aprotic polar compound in particular chosen from dimethyl sulphoxide (DMSO), diethyl sulphoxide, di-n-propyl sulphoxide, dimethylsulphone, sulpholane, dimethylformamide, dimethylacetamide, tetramethylurea and acetonitrile, or their derivatives.

8. Biological material according to one of Claims 1 to 7, **characterized in that** said transmembrane polypeptide is selected from the group consisting of a glycoprotein, a lipoprotein and a membrane receptor.

9. Biological material according to Claim 8, **characterized in that** said transmembrane polypeptide is selected from the group consisting of the rabies virus glycoprotein, gp160 and CD4.

10. Biological material according to any one of the previous Claims, **characterized in that** said target cell is a mammalian tumor cell, a mammalian cell infected with a viral pathogenic agent, or a mammalian cell infected with a bacterial pathogenic agent.

11. Biological material according to Claim 2, **characterized in that** it is in a form which allows the administration thereof to the body of a mammal, and optionally the prior culturing thereof.

12. Biological material according to Claim 11, **characterized in that** said target cells originate from the mammal to be treated.

13. Biological material according to Claim 11, **characterized in that** said target cells originate from a mammal other than the one to be treated and have undergone a treatment making them compatible.

14. Biological material according to one of Claims 1 to 13, **characterized in that** it also comprises at least one DNA sequence which ensures the expression of a compound which is involved in the activation of cytotoxic effector cells or of helper T lymphocytes.

15. Use of a biological material according to one of Claims 1 to 13 for preparing a pharmaceutical composition intended for treating or for preventing cancers or viral infections.

16. Use of a nucleic acid sequence comprising:
(i) a gene encoding an antibody or an antibody fragment fused with the transmembrane domain of a transmembrane polypeptide, and
(ii) elements providing the expression of said gene in vivo in target cells intended to be genetically modified by said nucleic acid sequence;
**characterized in that** said antibody or antibody fragment is capable of binding to a receptor which is selected from the group consisting of all or part of the TCR complex, for preparing pharmaceutical compositions intended for treating a mammal by gene transfer.

17. Pharmaceutical composition comprising a biological material according to one of Claims 1 to 14, advantageously in combination with a pharmaceutically acceptable vehicle.

18. Pharmaceutical composition according to Claim 17, comprising a biological material according to one of Claims 11 to 13 and a compound which is naturally responsible for the activation of cytotoxic effector cells or of helper T lymphocytes.

19. Pharmaceutical composition according to Claim 18, **characterized in that** said compound is a cytokine or a chemokine.

20. Non-germinal mammalian cell which does not naturally produce antibodies, **characterized in that** it is genetically modified with at least one nucleic acid sequence comprising:
(i) a gene encoding an antibody or an antibody fragment fused with the transmembrane domain of a transmembrane polypeptide, and
(ii) elements providing the expression of said gene in vivo in target cells intended to be genetically modified by said nucleic acid sequence;
**characterized in that** said antibody or antibody fragment- is capable of binding to a receptor which is selected from the group consisting of all or part of the TCR complex.

21. Method for the in vitro preparation of a cell according to Claim 20, **characterized in that** at least one nucleic acid sequence is introduced into a non-germinal mammalian cell which does not naturally produce antibodies, by any suitable means, said at least one nucleic acid sequence comprising:
(i) a gene encoding an antibody or an antibody fragment fused with the transmembrane domain of a transmembrane polypeptide, and
(ii) elements providing the expression of said gene in vivo in target cells intended to be genetically modified by said nucleic acid sequence;
**characterized in that** said antibody or antibody fragment is capable of binding to a receptor which is selected from the group consisting of all or part of the TCR complex, and then **in that**, from these cells, those which are genetically modified with said nucleic acid sequence are chosen.
